# EUROPEAN PATENT APPLICATION

(11) **EP 3 067 062 A1**
(43) Date of publication of application: **14.09.2016**
(21) Application number: 15290069.2
(22) Date of filing: 13.03.2015
(51) Int. Cl.: A61K 39/00, A61K 45/06, A61K 31/4704, A61P 35/00

(54) **COMBINATION OF TASQUINIMOD OR A PHARMACEUTICALLY ACCEPTABLE SALT THEREOF AND A PD1 AND/OR PDL1 INHIBITOR, FOR USE AS A MEDICAMENT**

(71) Applicant: IPSEN PHARMA S.A.S., 92100 Boulogne-Billancourt (FR)
(72) Inventor: The designation of the inventor has not yet been filed
(74) Representative: Audonnet, Nathalie

(57) **Abstract**

The present invention concerns a combination comprising tasquinimod, or a pharmaceutically acceptable salt thereof, and a PD1 and/or PDL1 inhibitor, for use as a medicament. It also concerns a pharmaceutical composition comprising tasquinimod, or a pharmaceutically acceptable salt thereof, and PD1 and/or PDL1 inhibitor.

## Description

The present invention concerns a combination comprising tasquinimod, or a pharmaceutically acceptable salt thereof, and a PD1 and/or PDL1 inhibitor, for use as a medicament. It also concerns a pharmaceutical composition comprising tasquinimod, or a pharmaceutically acceptable salt thereof, and a PD1 and/or PDL1 inhibitor.

### Background

Bladder cancer is any of several types of cancer arising from the epithelial lining (i.e. the urothelium) of the urinary bladder. Rarely the bladder is involved by non-epithelial cancers, such as lymphoma or sarcoma. It is a disease in which abnormal cells multiply without control in the bladder. The most common type of bladder cancer recapitulates the normal histology of the urothelium and is known as transitional cell carcinoma or more properly urothelial cell carcinoma. Five-year survival rates in the United States are around 77% (SEER Stat Fact Sheets: Bladder Cancer. NCI, Retrieved 18 June 2014). Bladder cancer is the 9^{th} leading cause of cancer with 430,000 new cases (World Cancer Report 2014. World Health Organization, 2014, pp. Chapter 1.1., ISBN 9283204298) and 165,000 deaths occurring in 2012.

There have been no major advances for the treatment of metastatic urothelial bladder cancer (UBC) in the last 30 years. Chemotherapy is still the standard of care. Patient outcomes, especially for those in whom chemotherapy is not effective or is poorly tolerated, remain poor (Choueiri, T. K. et al. Double-blind, randomized trial of docetaxel plus vandetanib versus docetaxel plus placebo in platinum-pretreated metastatic urothelial cancer. J. Clin. Oncol. 30, 507-512 (2012); Bellmunt, J. et al. Phase III trial of vinflunine plus best supportive care compared with best supportive care alone after a platinum-containing regimen in patients with advanced transitional cell carcinoma of the urothelial tract. J. Clin. Oncol., 27, 4454-4461(2009)).

One hallmark of UBC is the presence of high rates of somatic mutations (Cancer Genome Atlas Research Network., Comprehensive molecular characterization of " urothelial bladder carcinoma, Nature 507,315-322 (2014); Lawrence, M. S. et al., Mutational heterogeneity in cancer and the search for new cancer-associated genes, Nature 499, 214-218 (2013); Kandoth, C. et al. Mutational landscape and significance across 12 major cancer types, Nature 502,333-339 (2013)). These alterations may enhance the ability of the host immune system to recognize tumour cells as foreign owing to an increased number of antigens (Chen, D. S. & Mellman, I.Oncology meets immunology: the cancer-immunity cycle, Immunity39, 1-10 (2013)).

However, these cancers may also elude immune surveillance and eradication through the expression of programmed death-ligand 1 (PDL1; also called CD274 or B7-H1) in the tumour microenvironment (Chen et al., Molecular pathways: next-generation immunotherapy-inhibiting programmed death-ligand 1 and programmed death-1. Clin. Cancer Res., 18, 6580-6587 (2012); Van Rooij, N. et al., Tumor exome analysis reveals neoantigen-specific T-cell reactivity in an ipilimumab-responsive melanoma, J. Clin. Oncol. 31, e439-e442 (2013)).

PDL1 is a negative regulator of immune activation through inhibition of effective T cell function. The co-inhibitory receptor programmed death 1 (PD1) and its ligands are key regulators in a wide spectrum of immune responses and play a critical role in autoimmunity and self-tolerance as well as in cancer immunology. Emerging evidence suggests that cancer cells might use the PD1/PD-ligand (PDL) pathway to escape anti-tumor immunity. Based on this evidence, early phase human clinical trials targeting the PD1/PDL pathway are currently underway for multiple human cancers. An anti-PDL1 antibody is in phase II development for metastatic bladder cancer (Powles et al., MPDL3280A (anti-PDL1) treatment leads to clinical activity in metastatic bladder cancer, Nature. 2014 Nov 27;515 (7528):558-62. doi: 10.1038/nature13904.; Errico A., Immunotherapy: PD1-PDL1 axis: efficient checkpoint blockade against cancer, Nat Rev Clin Oncol. 2015 Feb;12(2):63. doi: 10.1038/nrclinonc.2014.221. Epub 2014 Dec 23.; Muenst Set al., The PD1/PDL1 pathway: biological background and clinical relevance of an emerging treatment target in immunotherapy., Expert Opin Ther Targets., 2015 Feb;19(2):201-11. doi: 10.1517/14728222.2014.980235., Epub 2014 Dec 10; M. S. Soloway. Intravesical and systemic chemotherapy in the management of superficial bladder cancer. Urol.Clin.North Am. 11 (4):623-635, 1984).

Tasquinimod, a quinoline-3-carboxyamide analog, inhibits the interaction of a protein called S100A9 with its different receptors (TLR4, RAGE, EMMPRIN). Tasquinimod has immunomodulatory, anti-angiogenic and anti-metastatic activities. By targeting S100A9, tasquinimod modulates suppressive myeloid cells, including Myeloid Derived Suppressive cells and macrophages (Shen Let al., Tasquinimod Modulates Suppressive Myeloid Cells and Enhances Cancer Immunotherapies in Murine Models., Cancer Immunol Res. 2014 Nov 4.; Jennbacken Ket al., Inhibition of metastasis in a castration resistant prostate cancer model by the quinoline-3-carboxamide tasquinimod (ABR-215050), Prostate. 2012 Jun 1; 72(8):913-24, doi: 10.1002/pros.21495. Epub 2011 Oct 5; Isaacs et al., Identification of ABR-215050 as lead second generation quinoline-3-carboxamide anti-angiogenic agent for the treatment of prostate cancer., Prostate. 2006 Dec 1; 66(16):1768-78). Tasquinimod is in phase III clinical development for prostate cancer and other solid tumors.

Tasquinimod targets the tumour microenvironment and modulates regulatory myeloid cell function, exerting immunomodulatory, anti-angiogenic and anti-metastatic properties. Tasquinimod may also suppress the tumour hypoxic response, contributing to its effects on the tumour microenvironment.

The inventors have now unexpectedly demonstrated that tasquinimod, or a pharmaceutically acceptable salt thereof, and a PD1 and/or PDL1 inhibitor block tumor proliferation in a synergistic manner and can be used together as a medicament, in particular for the treatment of cancer, and more particularly for the treatment of bladder cancer.

### Description of the invention

The invention thus relates to a combination comprising tasquinimod, or a pharmaceutically acceptable salt thereof, and a PD1 and/or PDL1 inhibitor, for use as a medicament.

In an embodiment, the PD1 and/or PDL1 inhibitor is selected from an antibody and a peptide.

The invention also relates to a combination comprising tasquinimod, or a pharmaceutically acceptable salt thereof, and a PD1 and/or PDL1 antibody, for use as a medicament.

The invention also relates to a combination comprising tasquinimod, or a pharmaceutically acceptable salt thereof, and a PD1 and/or PDL1 inhibitor that is not antibody, for use as a medicament. Preferably, the invention also relates to a combination comprising tasquinimod, or a pharmaceutically acceptable salt thereof, and a peptide as PD1 and/or PDL1 inhibitor, for use as a medicament.

The invention also relates to a combination comprising tasquinimod, or a pharmaceutically acceptable salt thereof, and a PD1 and/or PDL1 inhibitor, for use for the treatment of cancer.

The invention also relates to a combination comprising tasquinimod, or a pharmaceutically acceptable salt thereof, and a PD1 and/or PDL1 antibody, for use for the treatment of cancer.

The invention also relates to a combination comprising tasquinimod, or a pharmaceutically acceptable salt thereof, and a PD1 and/or PDL1 inhibitor that is not antibody, for use for the treatment of cancer. Preferably, the invention also relates to a combination comprising tasquinimod, or a pharmaceutically acceptable salt thereof, and a peptide as PD1 and/or PDL1, for use for the treatment of cancer.

In particular, said cancer is chosen from bladder cancer, melanoma, lung cancer such as NSCLC (Non Small Cell Lung Cancer), colorectal cancer, breast cancer, pancreatic cancer, prostate cancer, renal cell carcinoma, hematologic malignancies, in particular advanced hematologic malignancies, ovarian cancer, in particular, platinum-resistant ovarian cancer, neuroendocrine tumors (NET) and gastroenteropancreatic neuroendocrine tumors (GEP-NET). The cancer treated with the combination of the present invention can be any stage, e.g. early stage or late stage. In some embodiments, the treatment results in sustained response in the individual after cessation of the treatment. In some embodiments, the treatment produces a complete response, a partial response, or stable disease in the individual.

In a preferred embodiment, the invention also relates to a combination comprising tasquinimod, or a pharmaceutically acceptable salt thereof, and a PD1 and/or PDL1 inhibitor, in particular a PD1 and/or PDL1 antibody, for use for the treatment of cancer selected from bladder cancer, melanoma, lung cancer, prostate cancer, renal cell carcinoma, hematologic malignancies, in particular advanced hematologic malignancies, ovarian cancer, in particular, platinum-resistant ovarian cancer, neuroendocrine tumors (NET), in particular gastroenteropancreatic neuroendocrine tumors (GEP-NET).

In a preferred embodiment, the invention also relates to a combination comprising tasquinimod, or a pharmaceutically acceptable salt thereof, and a PD1 and/or PDL1 inhibitor, in particular a PD1 and/or PDL1 antibody, for use for the treatment of cancer selected from bladder cancer, prostate cancer and renal cell carcinoma.

More particularly, the combination for use as mentioned above is for the treatment of bladder cancer, in particular non muscle invasive bladder cancer, muscle invasive bladder cancer and metastatic and urethelial bladder cancer.

In one embodiment, the combination for use according to the invention improves progression-free survival and/or life expectancy.

In one embodiment of the combination according to the invention, tasquinimod, or a pharmaceutically acceptable salt thereof, and the PD1 and/or PDL1 inhibitor, are administered separately, sequentially or simultaneously.

In one embodiment of the combination according to the invention, tasquinimod, or a pharmaceutically acceptable salt thereof, and the PD1 and/or PDL1 antibody, are administered separately, sequentially or simultaneously.

In one embodiment of the combination according to the invention, tasquinimod, or a pharmaceutically acceptable salt thereof, and a peptide used as PD1 and/or PDL1 inhibitor, are administered separately, sequentially or simultaneously.

The present invention also relates to a pharmaceutical composition comprising tasquinimod, or a pharmaceutically acceptable salt thereof, and a PD1 and/or PDL1 inhibitor.

The present invention also relates to a pharmaceutical composition comprising tasquinimod, or a pharmaceutically acceptable salt thereof, and a PD1 and/or PDL1 antibody.

The present invention also relates to a pharmaceutical composition comprising tasquinimod, or a pharmaceutically acceptable salt thereof, and a peptide as PD1 and/or PDL1.

In particular, said pharmaceutical composition further comprises a pharmaceutically acceptable excipient.

The present invention also relates to products comprising tasquinimod, or a pharmaceutically acceptable salt thereof, and a PD1 and/or PDL1 inhibitor, in particular a PD1 and/or PDL1 antibody, as a combined preparation for simultaneous use; separate use or use spread out over time (i.e. sequential use) for the treatment of cancer, in particular bladder cancer. In another preferred embodiment, the present invention also relates to products comprising tasquinimod, or a pharmaceutically acceptable salt thereof, and a peptide as PD1 and/or PDL1 inhibitor, as a combined preparation for simultaneous use, separate use or use spread out over time for the treatment of cancer, in particular bladder cancer including e.g. non muscle invasive bladder cancer, muscle invasive bladder cancer and metastatic urothelial bladder cancer.

The present invention further relates to a method for treating cancer, in particular bladder cancer, comprising the administration, in a patient in need thereof, of a therapeutically effective amount of a combination comprising tasquinimod, or a pharmaceutically acceptable salt thereof, and a PD1 and/or PDL1 inhibitor, in particular a PD1 and/or PDL1 antibody or a peptide as PD1 and/or PDL1 inhibitor.

In another aspect, provided is a kit comprising tasquinimod and a PD1 and/or PDL1 inhibitor for treating or delaying progression of a cancer in an individual suffering from cancer. The kit may comprise a PD1 and/or PDL1 inhibitor and a package insert comprising instructions for using tasquinimod in combination with a PD1 and/or PDL1 inhibitor to treat or delay progression of cancer in an individual. The kit may also comprise a PD1 and/or PDL1 inhibitor and a package insert comprising instructions for using the PD1 and/or PDL1 inhibitor in combination with tasquinimod to treat or delay progression of cancer in an individual suffering from cancer. The kit may also comprise tasquinimod and a PD1 and/or PDL1 inhibitor and a package insert comprising instructions for using the tasquinimod and the PD1 and/or PDL1 inhibitor to treat or delay progression of cancer in an individual.

Tasquinimod, also known under 4-hydroxy-5-methoxy-N,1-dimethyl-2-oxo-N-[4-(trifluoromethyl)phenyl]-1,2-dihydroquinoline-3-carboxamide (CAS number 254964-60-8), is a compound of formula (I_{T}):

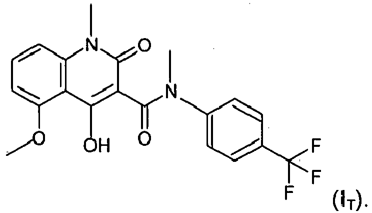

This compound, as well as tasquinimod-like compounds and their process of preparation are described in the patent application WO00/03991 (in particular in example 8), the content of which is incorporated by reference.

Tasquinimod-like compounds are e.g. compounds of general formula (I) 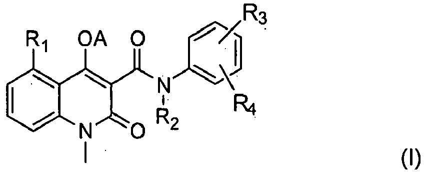 wherein
A is selected from hydrogen and -COR_{A} wherein A is selected from methyl, ethyl, n-propyl, iso-propyl, n-butyl, ter-butyl, neo-pentyl, phenyl, benzyl and phenethyl ;
R₂ is selected from methyl, ethyl, n-propyl, iso-propyl, n-butyl, iso-butyl, sec.butyl and allyl;
R₃ is selected from methyl, methoxy, fluoro, chloro, bromo, trifluoromethy), and -OCHₓF_{y}, wherein x = 0 - 2, y = 1 - 3 with the proviso that x + y = 3 and with the proviso that R' is nt hydrogen when R is methyl;
R₄ is selected from hydrogen, fluoro and chloro, with the proviso that R₄ is selected from fluoro and chloro only when R' is selected from fluoro and chloro;
R₁ is selected from methyl, ethyl, n-propyl, iso-propyl, methoxy, ethoxy, chloro, bromo, trifluoromethyl, dimethylamino, -OCHₓF_{y}, and -OCH₂CHₓF_{y} wherein x = 0 - 2, y = 1 - 3 with the proviso that x + y = 3;
or any tautomer and/or any pharmaceutically acceptable salts thereof.

Examples for such compounds are the following : N-ethyl-N-(3-fluoro-phenyl)-1,2-dihydro-4-hydroxy-5-chloro-1-methyl-2-oxo-quinoline-3-carboxamide ; N-methyl-N-(2,4-difluoro-phenyl)-1,2-dihydro-4-hydroxy-5-chloro-1-methyl-2-oxo-quinoline-3-carboxamide; N-methyl-N-(2,5-difluoro-phenyl)-1,2-dihydro-4-hydroxy-5-chloro-1-methyl-2-oxo-quinoline-3-carboxamide; N-ethyl-N-(3-methoxy-phenyl)-1,2-dihydro-4-hydroxy-5-ethyl-1-methyl-2-oxo-quinoline-3-carboxamide; N-methyl-N-(2,5-difluorophenyl)-1,2-dihydro-4-hydroxy-5-methoxy-1-methyl-2-oxo-quinoline-3-carboxamide; N-methyl-N-(4-trifluoromethyl-phenyl)-1,2-dihydro-4-hydroxy-5-methoxy-1-methyl-2-oxo-quinoline-3-carboxamide; or N-methyl-N-(2,4-difluoro-phenyl)-1,2-dihydro-4-hydroxy-5,6-methylenedioxy-1-methyl-2-oxo-quinoline-3-carboxamide.

The tasquinimod-like compounds are preferably compounds of formula (Ia) 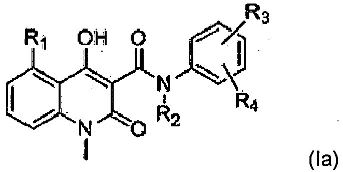 or a pharmaceutically acceptable salt thereof, wherein
R₁ is selected from H, methyl, ethyl, n-propyl, iso-propyl, methoxy, ethoxy, fluoro, chloro, bromo, trifluoromethyl, and trifluoromethoxy;
R₂ is C₁-C₄ alkyl;
R₃ is selected from methyl, methoxy, fluoro, chloro, bromo, trifluoromethyl, and trifluoromethoxy;
R₄ is selected from hydrogen, fluoro and chloro, with the proviso that R₄ is selected from fluoro and chloro only when R₃ is selected from fluoro and chloro; or a pharmaceutically acceptable salts thereof.

The term "C₁-C₄ alkyl" refers to a branched or unbranched alkyl group having from 1, 2, 3 or 4 carbon atoms, i.e. methyl, ethyl" n-propyl, isopropyl, n-butyl, *sec*-butyl, isobutyl or tert-butyl.

In some embodiments, R₁ is selected from methyl, ethyl, n-propyl, iso-propyl, methoxy, ethoxy, fluoro, chloro, bromo, trifluoromethyl, and trifluoromethoxy. In some other embodiments, R₁ is selected from ethyl, n-propyl, iso-propyl, methoxy, ethoxy, chloro, bromo, trifluoromethyl, and trifluoromethoxy. In still other embodiments, R₁ is selected from ethyl, methoxy, chloro, and trifluoromethyl. In some particular embodiments, R₁ is methoxy.

The moiety R₂ is a C₁-C₄ alkyl radical, which radical may be branched or linear. In some embodiments, R₂ is a C₁-C₃ alkyl radical. In some embodiments, R₂ is methyl or ethyl. In some particular embodiments, R₂ is methyl.

The moiety R₃ is selected from methyl, methoxy, fluoro, chloro, bromo, trifluoromethyl, and trifluoromethoxy. In some embodiments, R₃ is selected from methyl, methoxy, fluoro, chloro, trifluoromethyl, and trifluoromethoxy. In some particular embodiments, R₃ is trifluoromethyl.

R₄ is selected from hydrogen, fluoro and chloro, with the proviso that R₄ is selected from fluoro and chloro only when R₃ is selected from fluoro and chloro. In some, embodiments, R₄ is hydrogen or fluoro. In some particular embodiments, R₄ is hydrogen.

In some particular embodiments, in a compound of formula (Ia),
R₁ and R₄ are as defined herein above;
R₂ is methyl or ethyl, in particular methyl; and
R₃ is selected from methyl, methoxy, fluoro, chloro, trifluoromethyl, and trifluoromethoxy.

In some other particular embodiments, in a compound of formula (Ia),
R₁ is as defined herein above;
R₂ is methyl or ethyl, in particular methyl;
R₃ is selected from methyl, methoxy, fluoro, chloro, trifluoromethyl, and trifluoromethoxy; and R₄ is H.

In some embodiments, R₃ is in para-position, i.e. the compounds of formula (Ia) may be represented by formula (Ib) 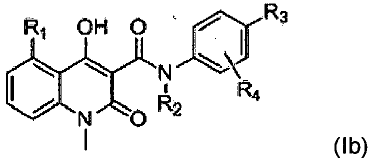 wherein R₁, R₂, R₃ and R₄ are as defined herein above.

For example, in some embodiments of a compound of formula (Ib),
R₁ and R₄ are as defined herein above;
R₂ is methyl or ethyl, in particular methyl; and
R₃ is selected from methyl, methoxy, fluoro, chloro, trifluoromethyl, and trifluoromethoxy.
In some other particular embodiments, in a compound of formula (Ib),
R₁ is as defined herein above;
R₂ is methyl or ethyl, in particular methyl;
R₃ is selected from methyl, methoxy, fluoro, chloro, trifluoromethyl, and trifluoromethoxy; and
R₄ is H.

As noted herein above, in some embodiments, R₄ is hydrogen. In those embodiments, the compound of formula (Ia) may be represented by formula (Ic) 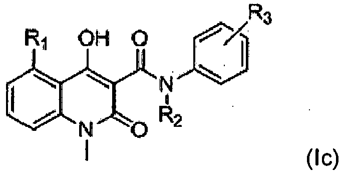 wherein R₁, R₂ and R₃ are as defined herein above.

For example, in some embodiments of a compound of formula (Ic),
R₂ is methyl or ethyl, in particular methyl;
R3 is selected from methyl, methoxy, fluoro, chloro, trifluoromethyl, and trifluoromethoxy;
and R₁ is as defined herein above.

In some particular embodiments of a compound of formula (Ia), R₃ is in para-position and R₄ is H, and the compound as defined herein may then be represented by formula (Id) 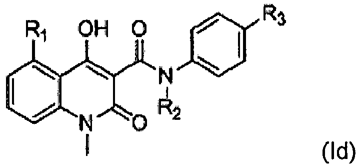 wherein R₁, R₂ and R₃ are as defined herein above.

In some particular embodiments of a compound of formula (Id), R₂ is methyl or ethyl, in particular methyl; R₃ is selected from methyl, methoxy, fluoro, chloro, trifluoromethyl, and trifluoromethoxy; and R₁ is as defined herein.

For the purpose of the present invention, any reference to a compound of formula (I) also should be understood as a reference to a compound of any one of the formulas (Ia), (Ib) (Ic) and (Id), unless otherwise specified or apparent from the context.

It is anticipated that any or all of the tasquinimod-like compounds as defined above in formula (I), (Ia), (Ib), (Ic) and (Id) exhibit a synergistic effect in combination with a PD1 and/or PDL1 inhibitor for treatment of cancer. Therefore, the invention also refers to a combination of a tasquinimod-like compound and a PD1 and/or PDL1 inhibitor for use as a medicament, particularly for use in cancer and more particularly for use in bladder cancer. All embodiments described herein for a combination of tasquinimod with a PD1 and/or PDL1 inhibitor equally apply to a combination of a tasquinimod-like compound and a PD1 and/or PDL1 inhibitor. Similarly, all embodiments described herein for pharmaceutical compositions, products, kits, methods of treatment and uses equally apply to a combination of a tasquinimod-like compound and a PD1 and/or PDL1 inhibitor.

By a "pharmaceutically acceptable salt" of tasquinimod or a tasquinimod-like compound, it is meant that the compound is modified by making acid or base salts thereof. The pharmaceutically acceptable salts include the conventional non-toxic salts or the quaternary ammonium salts of the compound, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propanoic, succinic, tartaric, citric, methanesulfonic, benzenesulfonic, glucuronic, glutamic, benzoic, salicylic, toluenesulfonic, oxalic, fumaric, maleic, and the like. Further addition salts include ammonium salts such as tromethamine, meglumine, epolamine, etc., metal salts such as sodium, potassium, calcium, zinc or magnesium. The pharmaceutically acceptable salts of tasquinimod can be synthesized from the parent compound by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418.

PD1 is a type I transmembrane protein of the immunoglobulin superfamily (Ishida et al., Induced expression of PD1, a novel member of the immunoglobulin gene superfamily, upon programmed cell death. EMBO J 1992; 11(11):3887-95), whose structure includes an extracellular domain, similar to the variable region of an immunoglobulin, followed by a transmembrane region and a cytoplasmic tail (Zhang et al., Structural and functional analysis of the costimulatory receptor programmed death-1. Immunity 2004; 20(3):337-4).

It binds two ligands, known as PDL1 (B7-H1, CD274) and PDL2 (B7-DC, CD273), which belong to the B7 protein family). PDL1 is also known as cluster of differentiation 274 (CD274) or B7 homolog 1 (B7-H1) and is a protein that in humans is encoded by the CD274 gene. PDL1 is a 40kDa type 1 transmembrane protein that has been speculated to play a major role in suppressing the immune system during particular events such as pregnancy, tissue allografts, autoimmune disease and other disease states such as hepatitis. Normally the immune system reacts to foreign antigens where there is some accumulation in the lymph nodes or spleen which triggers a proliferation of antigen-specific CD8+ T cell. The formation of PD1 receptor/PDL1 or B7.1 receptor PDL1 ligand complex transmits an inhibitory signal which reduces the proliferation of these CD8+ T cells at the lymph nodes and supplementary to that PD1 is also able to control the accumulation of foreign antigen specific T cells in the lymph nodes through apoptosis which is further mediated by a lower regulation of the gene Bcl-2 (Chemnitz et al., (July 2004)., SHP-1 and SHP-2 associate with immunoreceptor tyrosine-based switch motif of programmed death 1 upon primary human T cell stimulation, but only receptor ligation prevents T cell activation, Journal of Immunology 173 (2): 945-54).

By "PD1 inhibitor" is meant any inhibitor or antagonist of the biological effect of PD1, i.e. a molecule that blocks the interaction between PD1 and its ligands, PDL1 and PDL2.

By "PDL1 inhibitor" is meant any inhibitor or antagonist of the biological effect of PDL1, i.e. a molecule that blocks the interaction between PDL1 and its receptor or receptors, e.g. PD1 and/or B7.1 (CD80).

A PD1 and/or PDL1 inhibitor can e.g. be a small molecule, a peptide, protein, an antibody, an antagonistic nucleic acid such as a siRNA, miRNA, antisense RNA or the like.

In some embodiments of the present invention, in the combination for use according to the invention, or pharmaceutical composition according to the invention, or products or kits according to the invention, or methods according to the invention, the PD1 and/or PDL1 inhibitor is selected from an antibody or a peptide.

The person skilled in the art knows how to determine whether a compound is a PD1 and/or PDL1 inhibitor by testing it in an appropriate assay. Binding of inhibitors to PD1 and/or PDL1 and/or PDL2 can e.g. be measured in ELISA-type assays that are well known in the art. Bioassays to measure the biological effect of PD1 and/or PDL1 and/or PDL2 inhibition are well known by the person skilled in the art. For instance, an assay called "mixed lymphocyte reaction (MLR) assay has been described (Poster LB-266, presented at the 2014 conference of the American Association of Cancer Research, AACR 2014). In this assay, isolated peripheral blood monocytes from a human donor are differentiated into dendritic cells (DCs) and then mixed with CD4⁺ T-cells isolated from a second donor. IL-2 levels are meatured after 48 hours in the presence or absence of increasing concentrations of a PD1 and/or PDL1 inhibitor and compared to an appropriate control such as an isotype control if the inhibitor is an antibody).

Examples for suitable PDL1 inhibitors that are peptides are e.g. the peptides described in WO 2013/144704, in particular a peptide called AUR-012, which is described in Proceedings: AACR Annual Meeting 2014; April 5-9, 2014; San Diego, CA, and that antagonizes both PDL1 and PLD2 activities.

By "PD1 and/or PDL1 antibody", it is meant an antibody able to block the PD1 and/or PDL1 pathway, preferably by preventing the binding between PD1 and PDL1. Such antibodies are known by the person skilled in the art. Examples can be nivolumab, also known as ONO-4538, BMS-936558 or MDX1106 (Bristol-Myers Squibb), pembrolizumab, also known as MK-3475 (Merck & Co.), pidilizumab, also known as CT-011 (CureTech) or e.g. further antibodies such as BMS936559 or MPDL3280A.

In some embodiments, the PD1 and/or PDL1 inhibitor is an anti-PD1 antibody (e.g., a human antibody, a humanized antibody, or a chimeric antibody). In some embodiments, the anti-PD1 antibody is selected from the group consisting of nivolumab (MDX-1106), pembrolizumab (Merck 3475) and CT-011. In some embodiments, the PD1 and/or PDL1 inhibitor is an immunoadhesin such as e.g. an immunoadhesin comprising an extracellular or PD1 binding portion of PDL1 or PDL2 fused to a constant region such as an Fc region of an immunoglobulin sequence. In some embodiments, the PD1 and/or PDL1 inhibitor is AMP-224. In some embodiments, the PDL1 binding antagonist is anti-PDL1 antibody. In some embodiments, the PD1 and/or PDL1 inhibitor is selected from the group consisting of YW243.55.S70, MPDL3280A and MDX-1105. MDX-1105, also known as BMS-936559, is an anti-PDL1 antibody described in WO2007/005874. Antibody YW243.55.S70 is an anti-PDL1 described in WO 2010/077634. MDX-1106, also known as MDX-1106-04, ONO-4538 or BMS-936558, is an anti-PD 1 antibody described in WO2006/121168. Merck 3745, also known as MK-3475 or SCH-900475, is an anti-PD1 antibody described in WO2009/114335. CT-011, also known as hBAT or hBAT-1 , is an anti-PD1 antibody described in WO2009/10161 1. AMP-224, also known as B7-DClg, is a PDL2-Fc fusion soluble receptor described in WO2010/027827 and WO2011/066342.

In an embodiment of the present invention, in the combination for use according to the invention, or pharmaceutical composition according to the invention, or products according to the invention, or methods according to the invention, the preferred PD1 and/or PDL1 antibodies are pembrolizumab and nivolumab.

In an embodiment of the present invention, the combination for use according to the invention, or the pharmaceutical composition according to the invention, or products according to the invention, or methods according to the invention, comprises tasquinimod, or any pharmaceutically acceptable salts thereof, and pembrolizumab as PD1/PDL1 antibody.

In an embodiment of the present invention, the combination for use according to the invention, or the pharmaceutical composition according to the invention, or products according to the invention, or methods according to the invention, comprises tasquinimod, or any pharmaceutically acceptable salts thereof, and pidilizumab as PD1/PDL1 antibody.

In an embodiment of the present invention, the combination for use according to the invention, or the pharmaceutical composition according to the invention, or products according to the invention, or methods according to the invention, comprises tasquinimod, or any pharmaceutically acceptable salts thereof, and nivolumab as PD1/PDL1 antibody.

In an embodiment of the present invention, the combination for use according to the invention, or the pharmaceutical composition according to the invention, or products according to the invention, or methods according to the invention, comprises tasquinimod, or any pharmaceutically acceptable salts thereof, and MPDL3280A as PD1/PDL1 antibody.

In an embodiment of the present invention, the combination for use according to the invention, or the pharmaceutical composition according to the invention, or products according to the invention, or methods according to the invention, comprises tasquinimod, or any pharmaceutically acceptable salts thereof, and BMS 936559 as PD1/PDL1 antibody.

In an embodiment of the present invention, the combination for use according to the invention, or the pharmaceutical composition according to the invention, or products according to the invention, or methods according to the invention, comprises tasquinimod, or any pharmaceutically acceptable salts thereof, and AUR-012 as PD1/PDL1 inhibitor.

An "antibody" may be a natural or conventional antibody in which two heavy chains are linked to each other by disulfide bonds and each heavy chain is linked to a light chain by a disulfide bond. There are two types of light chain, lambda (I) and kappa (k). There are five main heavy chain classes (or isotopes) which determine the functional activity of an antibody molecule: IgM, IgD, IgG, IgA and IgE, having heavy chains designated α, δ, ε, γ and µ, respectively. The γ and α classes are further divided into subclasses on the basis of relatively minor differences in the CH sequence and function, e.g., humans express the following subclasses: IgG1, IgG2A, IgG2B, IgG3, IgG4, IgA1 and IgA2. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region.

"Framework Regions" (FRs) refer to amino acid sequences interposed between CDRs, *i.e.* to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively.

As used herein, a "human framework region" is a framework region that is substantially identical (about 85%, or more, in particular 90%, 95%, 97%, 99% or 100%) to the framework region of a naturally occurring human antibody.

As used herein, the term "antibody" denotes conventional antibodies and fragments thereof, as well as single domain antibodies and fragments thereof, in particular variable heavy chain of single domain antibodies, and chimeric; humanized, bispecific or multispecific antibodies.

As used herein, antibody or immunoglobulin also includes "single domain antibodies" which have been more recently described and which are antibodies whose complementary determining regions are part of a single domain polypeptide. Examples of single domain antibodies include heavy chain antibodies, antibodies naturally devoid of light chains, single domain antibodies derived from conventional four-chain antibodies, engineered single domain antibodies. Single domain antibodies may be derived from any species including, but not limited to mouse, human, camel, llama, goat, rabbit and bovine. Single domain antibodies may be naturally occurring single domain antibodies known as heavy chain antibody devoid of light chains. In particular, *Camelidae* species, for example came, dromedary, llama, alpaca and guanaco, produce heavy chain antibodies naturally devoid of light chain. Camelid heavy chain antibodies also lack the CH1 domain.

The variable heavy chain of these single domain antibodies devoid of light chains are known in the art as "VHH" or "nanobody". Similar to conventional VH domains, VHHs contain four FRs and three CDRs. Nanobodies have advantages over conventional antibodies: they are about ten times smaller than IgG molecules, and as a consequence properly folded functional nanobodies can be produced by *in vitro* expression while achieving high yield. Furthermore, nanobodies are very stable, and resistant to the action of proteases. The properties and production of nanobodies have been reviewed by Harmsen and De Haard HJ (Appl. Microbiol. Biotechnol. 2007 Nov; 77(1): 13-22).

The antibody of the invention may be a polyclonal antibody or a monoclonal antibody. Said monoclonal antibody may be humanized. In another example the antibody may be a fragment selected from the group consisting of Fv, Fab, F(ab')2, Fab', dsFv, (dsFv)2, scFv, sc(Fv)2, diabodies and VHH.

The term "monoclonal antibody" or "mAb" as used herein refers to an antibody molecule of a single amino acid composition that is directed against a specific antigen, and is not to be construed as requiring production of the antibody by any particular method. A monoclonal antibody may be produced by a single clone of B cells or hybridoma, but may also be recombinant, i.e. produced by protein engineering.

The term "chimeric antibody" refers to an engineered antibody which in its broadest sense contains one or more regions from one antibody and one or more regions from one or more other antibody(ies). In particular, a chimeric antibody comprises a VH domain and a VL domain of an antibody derived from a non-human animal, in association with a CH domain and a CL domain of another antibody, in particular a human antibody. As the non-human animal, any animal such as mouse, rat, hamster, rabbit or the like can be used. A chimeric antibody may also denote a multispecific antibody having specificity for at least two different antigens. In an embodiment, a chimeric antibody has variable domains of mouse origin and constant domains of human origin.

The term "humanized antibody" refers to an antibody which is initially wholly or partially of non-human origin and which has been modified to replace certain amino acids, in particular in the framework regions of the heavy and light chains, in order to avoid or minimize an immune response in humans. The constant domains of a humanized antibody are most of the time human CH and CL domains. In an embodiment, a humanized antibody has constant domains of human origin.

"Fragments" of antibodies comprise a portion of an intact antibody, in particular the antigen binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of an antibody may also be a single domain antibody, such as a heavy chain antibody or VHH.

The term "Fab" denotes an antibody fragment having a molecular weight of about 50,000 Da and antigen binding activity, in which about a half of the N-terminal side of H chain and the entire L chain, among fragments obtained by treating IgG with a protease, papaine, are bound together through a disulfide bond.

The term "F(ab')2" refers to an antibody fragment having a molecular weight of about 100,000 Da and antigen binding activity, which is slightly larger than the Fab bound via a disulfide bond of the hinge region, among fragments obtained by treating IgG with a protease, pepsin.

The term "Fab' " refers to an antibody fragment having a molecular weight of about 50,000 Da and antigen binding activity, which is obtained by cutting a disulfide bond of the hinge region of the F(ab')2.

A single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)₂. "dsFv" is a VH::VL heterodimer stabilised by a disulphide bond.

"(dsFv)2" denotes two dsFv coupled by a peptide linker.

The term "bispecific antibody" or "BsAb" denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term "multispecific antibody" denotes an antibody which combines the antigen-binding sites of two or more antibodies within a single molecule.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

Typically, antibodies are prepared according to conventional methodology. Monoclonal antibodies may be generated using the method of Kohler and Milstein (Nature, 256:495, 1975). To prepare monoclonal antibodies useful in the invention, a mouse or other appropriate host animal is immunized at suitable intervals (e.g., twice-weekly, weekly, twice-monthly or monthly) with the relevant antigenic forms. The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The animals may be immunized by subcutaneous, intraperitoneal, intramuscular, intravenous, intranasal or other routes. A given animal may be immunized with multiple forms of the antigen by multiple routes.

This invention provides in certain embodiments compositions and methods that include humanized forms of antibodies. Methods of humanization include, but are not limited to, those described in U.S. Pat. Nos. 4,816,567, 5,225,539, 5,585,089, 5,693,761, 5,693,762 and 5,859,205, which are hereby incorporated by reference. The above U.S. Pat. Nos. 5,585,089 and 5,693,761, and WO 90/07861 also propose four possible criteria which may be used in designing the humanized antibodies. The first proposal was that for an acceptor, use a framework from a particular human immunoglobulin that is unusually homologous to the donor immunoglobulin to be humanized, or use a consensus framework from many human antibodies. The second proposal was that if an amino acid in the framework of the human immunoglobulin is unusual and the donor amino acid at that position is typical for human sequences, then the donor amino acid rather than the acceptor may be selected. The third proposal was that in the positions immediately adjacent to the 3 CDRs in the humanized immunoglobulin chain, the donor amino acid rather than the acceptor amino acid may be selected. The fourth proposal was to use the donor amino acid reside at the framework positions at which the amino acid is predicted to have a side chain atom within 3A of the CDRs in a three dimensional model of the antibody and is predicted to be capable of interacting with the CDRs. The above methods are merely illustrative of some of the methods that one skilled in the art could employ to make humanized antibodies. One of ordinary skill in the art will be familiar with other methods for antibody humanization.

In one embodiment of the humanized forms of the antibodies, some, most or all of the amino acids outside the CDR regions have been replaced with amino acids from human immunoglobulin molecules but where some, most or all amino acids within one or more CDR regions are unchanged. Small additions, deletions, insertions, substitutions or modifications of amino acids are permissible as long as they would not abrogate the ability of the antibody to bind a given antigen. Suitable human immunoglobulin molecules would include IgGI, IgG2, IgG3, IgG4, IgA and IgM molecules. A "humanized" antibody retains a similar antigenic specificity as the original antibody. However, using certain methods of humanization, the affinity and/or specificity of binding of the antibody may be increased using methods of "directed evolution", as described by Wu et al., /. Mol. Biol. 294:151, 1999, the contents of which are incorporated herein by reference.

Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein, the contents of which are incorporated herein by reference. These animals have been genetically modified such that there is a functional deletion in the production of endogenous (e.g., murine) antibodies. The animals are further modified to contain all or a portion of the human germ-line immunoglobulin gene locus such that immunization of these animals will result in the production of fully human antibodies to the antigen of interest. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (KAMA) responses when administered to humans.

*In vitro* methods also exist for producing human antibodies. These include phage display technology (U.S. Pat. Nos. 5,565,332 and 5,573,905) and *in vitro* stimulation of human B cells (U.S. Pat. Nos. 5,229,275 and 5,567,610). The contents of these patents are incorporated herein by reference.

In one embodiment, the antibody of the invention is modified to reduce or inhibit the ability of the antibody to mediate antibody dependent cellular cytotoxicity (ADCC) and/or complement dependent cytotoxicity (CDC) functionality (i.e. an antibody with reduced Fc-effector function"). In particular, the antibodies of the present invention have no Fc portion or have an Fc portion that does not bind FcyRI and C1q. In one embodiment, the Fc portion of the antibody does not bind FcyRI, C1q, or FcyRIII. Antibodies with such functionality, in general, are known. There are native such antibodies, such as antibodies with an IgG4 Fc region. There also are antibodies with Fc portions genetically or chemically altered to eliminate the Antibody dependent cell cytotoxicity (ADCC) and/or complement dependent cytotoxicity (CDC) functionality.

The terms "treat", "treating", "treated" or "treatment", as used herein, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition.

As used herein and unless otherwise defined, "cancer" refers to the growth, division or proliferation of abnormal cells in the body. Cancers that can be treated with the combinations, pharmaceutical compositions, products and methods described herein include, but are not limited to, bladder cancer, melanoma, lung cancer such as NSCLC (Non Small Cell Lung Cancer), colorectal cancer, breast cancer, pancreatic cancer, renal cell carcinoma, hematologic malignancies, in particular advanced hematologic malignancies, ovarian cancer, in particular, platinum-resistant ovarian cancer, neuroendocrine tumors (NET) and gastroenteropancreatic neuroendocrine tumors (GEP-NET). In particular and as previously mentioned, one of the cancers that can be treated in accordance with the present invention is bladder cancer, for example non muscle invasive bladder cancer, muscle invasive bladder cancer and metastatic urothelial bladder cancer.

As shown in Example 2 below, data from an experimental tumor model indicate that treatment of cancer with tasquinimod may increase PDL1 levels on myeloid cells, which may attenuate the antitumor effect of tasquinimod. Adding a PD1/PDL1 inhibitor to tasquinimod can therefore potentiate the antitumor activity of tasquinimod.

In view of this data, the invention also relates to a method of treating a patient suffering from a cancer in which cancer cells express a detectable or increased level of PD1 and/or PDL1 and/or PDL2 by administering a therapeutically effective amount of a combination of tasquinimod and a PD1 and/or PLD1 inhibitor to the patient in need thereof.

In one embodiment, the patient is first treated with tasquinimod for a first period, followed by treatment with the combination according to the present invention for a second period. The first period can e.g. be a period during which cancer cells from the patient do not express detectable levels of PD1 and/or PDL1 and/or PDL2. The second period can e.g. be a period during which cancer cells from the patient express detectable levels of PD1 and/or PDL1 and/or PDL2.

As a consequence, the present invention also relates to a combination for use according to the invention, or pharmaceutical composition according to the invention, or products (including kits) according to the invention, for use in the treatment of a cancer in which cancer cells express a detectable or increased level of PD1 and/or PDL1 and/or PDL2. For instance the combination of the invention can be used for treatment of bladder cancer, in which cancer cells express detectable levels of PDL1.

The invention further relates to a method for identifying a patient suffering from a cancer, e.g. a bladder cancer, who will most likely benefit from a treatment with a combination comprising tasquinimod, or a pharmaceutically acceptable salt thereof, and a PD1 and/or PDL1 inhibitor, said method comprising:
i) in a sample obtained from the patient, determining the level of expression of PD1 or PDL1 or PDL2 expressed by cancer cells;
wherein a detectable or increased level of PD1 or PDL1 or PDL2 in the sample obtained from the patient compared to a reference value indicates that the patient will most likely benefit from a treatment with said combination.

The level of expression of the PD1 or PDL1 or PDL2 may be, for instance, determined by any method known to the skilled person, for example by RNA expression analysis by PCR such as e.g. quantitative real-time PCR, or by an immunoassay such as e.g. an enzyme-linked immunosorbent assay (ELISA) or by flow cytometry (FACS) analysis analytical methods, like for example mass spectrometry (MS), capillary electrophoresis-mass spectrometry (CE-MS), liquid chromatography coupled to mass spectrometry (LC-MS, LC-MS/MS).

The term "immunoassay" as used according to the present invention includes competition, direct reaction, or sandwich type assays. Such assays include, but are not limited to, agglutination test, enzyme-labelled and mediated immunoassays, such as ELISA, biotin/avidin type assay, radioimmunoassay, immunoelectrophoresis, and immunoprecipitation, and is more directly related to ELISA.

Mass spectrometry (MS), capillary electrophoresis-mass spectrometry (CE-MS), liquid chromatography coupled to mass spectrometry (LC-MS/MS), are all analytical methods very well known by the person skilled in the art.

The techniques cited above are described as appropriate methods for measuring the level of PD1 or PDL1 or PDL2, but the scope of the invention is not limited by these examples. Any other method known by the person skilled in the art to detect the level of expression of PD1 or PDL1 or PDL2 can be used.

As used herein, the term "sample" means a substance of biological origin. Examples of biological samples include, but are not limited to blood, plasma, serum, biopsy or saliva. The biological sample according to the invention may be obtained from the subject by any appropriate means of sampling known from the person skilled in the art.

For determining the level of expression of PD1 or PDL1 or PDL2 in cancer cells, the sample is preferably a biopsy of tumor, e.g. of a bladder tumor.

Preferably the reference value is measured in a sample from the same tissue origin as the sample of the patient of step i) of the above mentioned method.

Preferably, the "reference value" corresponds to the normal level of PD1 or PDL1 or PDL2.

As intended herein a "normal level" of PD1 or PDL1 or PDL2 means that the level of PDL1 in the sample is within the norm cut-off values for PD1 or PDL1 or PDL2. The norm is dependent on the sample type and on the method used for measuring the level of PD1 or PDL1 or PDL2 in the sample. In particular, the reference value of PD1 or PDL1 or PDL2 may thus correspond to the absence of, or to a basal level of expression of PD1 or PDL1 or PDL2 in normal cells.

A detectable level of expression is considered to be statistically significant if the level of expression of PD1 or PDL1 or PDL2 in biological sample of the patient is increased from undetectable to detectable levels of PD1 or PDL1 or PDL2. An increased level of expression is considered to be statistically significant if the level of expression of PD1 or PDL1 or PDL2 in biological sample of the patient is increased by order at least 10 or 15 or 20 or 25 or 30 or 35 or 40 or 45 or 50% compared with the reference value of level of expression

As used herein, the term "patient" refers to a warm-blooded animal such as a mammal, in particular a human, male or female, which is afflicted with, or has the potential to be afflicted with one or more diseases and conditions described herein.

As used herein, a "therapeutically effective amount" refers to an amount which is effective in reducing, eliminating, treating or controlling the symptoms of the herein-described diseases and conditions. An "effective amount" is at least the minimum concentration required to effect a measurable improvement or prevention of a particular disorder. An effective amount herein may vary according to factors such" as the disease state, age, sex, and weight of the patient, and the ability of the antibody to elicit a desired response in the individual. An effective amount is also one in which any toxic or detrimental effects of the treatment are outweighed by the therapeutically beneficial effects. For prophylactic use, beneficial or desired results include results such as eliminating or reducing the risk, lessening the severity, or delaying the onset of the disease, including biochemical, histological and/or behavioral symptoms of the disease, its complications and intermediate pathological phenotypes presenting during development of the disease. For therapeutic use, beneficial or desired results include clinical results such as decreasing one or more symptoms resulting from the disease, increasing the quality of life of those suffering from the disease, decreasing the dose of other medications required to treat the disease, enhancing effect of another medication such as via targeting, delaying the progression of the disease, and/or prolonging survival. In the case of cancer or tumor, an effective amount of the drug may have the effect in reducing the number of cancer cells; reducing the tumor size; inhibiting (i.e., slowing to some extent or desirably stopping) cancer cell infiltration into peripheral organs; inhibit (i.e., slowing to some extent and desirably stopping) tumor metastasis; inhibiting to some extent tumor growth; and/or relieving to some extent one or more of the symptoms associated with the disorder. An effective amount can be administered in one or more administrations. For purposes of this invention, an effective amount of drug, compound, or pharmaceutical composition or combination thereof, is an amount sufficient to accomplish prophylactic or therapeutic treatment either directly or indirectly. An "effective amount" in the context of administering more than one therapeutic agents, also refers to the amount of a single agent if, in conjunction with one or more other agents, a desirable result may be or is achieved.

The term "controlling" is intended to refer to all processes wherein there may be a slowing, delaying, interrupting, arresting, or stopping of the progression of the diseases and conditions described herein, but does not necessarily indicate a total elimination of all disease and condition symptoms, and is intended to include prophylactic treatment and chronic use.

As used herein, a "pharmaceutically acceptable excipient" refers to molecular entities and compositions that do not produce an adverse, allergic or other untoward reaction when administered to a mammal, especially a human, as appropriate. A pharmaceutically acceptable excipient refers to a non-toxic solid, semi-solid or liquid filler, diluent, encapsulating material or formulation auxiliary of any type.

In one embodiment, the combination for use according to the invention, or pharmaceutical composition according to the invention, or products according to the invention, are used to treat bladder cancer in patients in whom chemotherapy is not yet clinically indicated.

The present invention thus also relates to a combination for use according to the invention, or pharmaceutical composition according to the invention, or products according to the invention, for use in the treatment of cancer, in particular bladder cancer, as a 1^{st} line treatment.

It also relates to a method of treatment according to the invention, wherein the combination comprising tasquinimod, or a pharmaceutically acceptable salt thereof, and a PD1 and/or PDL1 inhibitor, in particular an antibody, is a 1^{st} line treatment.

By "1^{st} line treatment" or "1^{st} line therapy" is meant, in the context of the invention, the first treatment given to treat a cancer, in particular bladder cancer. It can be part of a standard set of treatments, such as surgery and radiation. It can also be used by itself. It can also be called induction therapy or primary therapy.

In another embodiment, the combination for use according to the invention, or pharmaceutical composition according to the invention, or products according to the invention, are used to treat a cancer, in particular bladder cancer in patients in whom other therapies have been indicated as previous treatments. Such therapies include surgery, chemotherapy such as mitomycin C, cisplatin-based chemotherapy, gemcitabine plus cisplatin or methotrexate, vinflunine, vinblastine or doxorubicine, or radiation therapy.

The present invention thus also relates to a combination for use according to the invention, or pharmaceutical composition according to the invention, or products according to the invention, for use in the treatment of cancer, in particular bladder cancer, as a 2^{nd}, 3^{rd}, or 4^{th} line treatment.

It also relates to a method of treatment according to the invention, wherein the combination comprising tasquinimod, or a pharmaceutically acceptable salt thereof, and a PD1 and/or PDL1 antibody, is a 2^{nd}, 3^{rd}, or 4^{th} line treatment.

By "2^{nd} line treatment" or "2^{nd} line therapy" is meant, the treatment that is given when initial treatment such as chemotherapeutic treatment (1^{st} line treatment) doesn't work, or stops working.

By "3^{rd} line treatment" or "3^{rd} line therapy" is meant the treatment that is given when both initial treatment such chemotherapeutic treatment (1^{st} line treatment) and subsequent treatment (2^{nd} line treatment) don't work, or stop working.

By "4^{th} line treatment" or "4^{th} line therapy" is meant the treatment that is given when both initial treatment such as chemotherapeutic treatment (1^{st} line treatment) and subsequent treatment (2^{nd} and 3^{rd} line treatment) don't work, or stop working.

In one embodiment, the combination for use according to the invention consists of tasquinimod, or a pharmaceutically acceptable salt thereof, and a PD1/PDL1PD1 and/or PDL1 antibody, in particular Pembrolizumab (Keytruda®, MK-3475, Merck) or Nivolumab (Opdivo, Bristol-Myers Squibb).

Compounds of the combinations, pharmaceutical compositions and products of the invention may be prepared by a variety of synthetic routes. The reagents and starting materials are commercially available, or readily synthesized by well-known techniques by the person skilled in the art.

The identification of the subjects who are in need of treatment of herein-described diseases and conditions is well within the ability and knowledge of the person skilled in the art. A clinician skilled in the art can readily identify, by the use of clinical tests, physical examination and medical/family history, those subjects who are in need of such treatment.

A therapeutically effective amount can be readily determined by the attending physician or diagnostician, as one skilled in the art, by the use of conventional techniques and by observing results obtained under analogous circumstances. In determining the therapeutically effective amount, a number of factors are considered by the attending physician or diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances. The amount of each compound of the combinations, pharmaceutical compositions and products of the invention, which are required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

In particular, the combinations for use according to the invention, or pharmaceutical compositions according to the invention, or products according to the invention, comprise the dose known as active for the PD1 and/or PDL1 inhibitor in the disease to be treated, in cancer in particular.

In particular, the combinations for use according to the invention, or pharmaceutical compositions according to the invention, or products according to the invention, comprise from 0.001 to 50, more particularly from 0.01 to 20, and in a preferred embodiment from 0.1 to 5 mg/day of tasquinimod or a pharmaceutically acceptable salt thereof.

More particularly, the combinations for use according to the invention, or pharmaceutical compositions according to the invention, or products according to the invention, comprise from 0.1 to 5 mg/day of tasquinimod or a pharmaceutically acceptable salt thereof and a dose corresponding to the active dose of the PD1 and/or PDL1 inhibitor in the disease to be treated, and in cancer in particular.

Each compound of the combinations according to the invention can be administered separately, sequentially or simultaneously. If administered simultaneously, the compounds of the combinations of the invention can e.g. be formulated in a single pharmaceutical composition. Alternatively, each compound can be formulated and administered independently.

Accordingly, the invention further relates to a combination for use as defined above, wherein tasquinimod, or a pharmaceutically acceptable salt thereof, and a PD1 and/or PDL1 inhibitor, are administered separately, sequentially or simultaneously.

If administered sequentially, the order of administration is flexible. For example, the PD1 and/or PDL1 inhibitor can be administered prior to administration of tasquinimod, or a pharmaceutically acceptable salt thereof. Alternatively, administration of tasquinimod, or a pharmaceutically acceptable salt thereof can precede administration of the PD1 and/or PDL1 inhibitor.

If administered separately, they can be administered by the same or different modes of administration. Examples of modes of administration include parenteral e.g., subcutaneous, intramuscular, intravenous, intradermal, as well as oral administration. In an embodiment of the invention, the PD1 and/or PDL1 inhibitor is administered parenterally and tasquinimod is administered orally.

In another embodiment, combinations for use according to the invention, or pharmaceutical compositions according to the invention, or products according to the invention, are intended for oral administration.

Whether the compounds of the combinations, pharmaceutical compositions and products as defined above are administered separately, sequentially or simultaneously, the compositions, combinations and products can take various forms. They may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter.

Compounds provided herein can be formulated into pharmaceutical compositions by admixture with one or more pharmaceutically acceptable excipients. Such compositions may be prepared for use in oral administration, particularly in the form of tablets or capsules, in particular orodispersible (lyoc) tablets; or parenteral administration, particularly in the form of liquid solutions, suspensions or emulsions.

In particular, the PD1 and/or PDL1 antibody is formulated in a form for injection In particular, the PD1 and/or PDL1 antibody is administered every two or three week. For instance, pembrolizumab is formulated in a lyophilized powder for injection and administered at the dose of 2 mg/kg every three weeks. Nivolumab is formulated in a liquid form for injection and administered at the dose of 3 mg/kg every two weeks.

In particular, tasquinimod or a pharmaceutically acceptable salt thereof, is formulated in the form of a tablet or hard gelatin capsules, more particularly comprising from 0.1 to 10 mg, even more particularly from 0.2 to 1.5 mg of tasquinimod or a pharmaceutically acceptable salt thereof and for example in a tablet of 0.25, 0.5 or 1 mg of said compound. In particular, tasquinimod or a pharmaceutically acceptable salt thereof is administered twice daily.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally.made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

For example, tasquinimod or a pharmaceutically acceptable salt thereof can be formulated in the form of a tablet or hard gelatin capsules.

For example, the PD1 and/or PDL1 inhibitor can be formulated in the form of liquid or lyophilized powder reconstituted and/or diluted for an administration by injection.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

The present invention will be further illustrated by the following figure and example.

### Brief description of the drawings

Figure 1: The combination of tasquinimod and a PD1/PDL1 antibody synergistically amplifies the antitumor effect in comparison to monotherapy
Figure 2: Gene expression profiling in MBT-2 tumors derived from mice treated with 30 mg/kg p.o. of tasquinimod twice daily from day 10 after tumor inoculation.
Tasquinimod treatment was carried out for 10 days. Data are represented as fold change of treated tumors compared to control tumors.
Figure 3: Median Fluorescence Intensity (MFI) of PD-L1 in CD11b+ cells derived from mice bearing tumors treated with tasquinimod. The mice were treated with 30 mg/kg p.o. of tasquinimod twice daily from day 10 after tumor inoculation. Tasquinimod treatment was carried out for 7 days. This analysis was done by cytometry.

### Example 1: Synergistic effect of the combination of tasquinimod and a PD1/PDL1 antibody on the reduction of tumor volume in a cancer mouse model

### Experimental Procedures

### Mice and cell line

Six to seven-week old C3H/HeN male mice were purchased from JANVIER Labs. MBT-2, a mouse bladder cancer cell line (M. S. Soloway., Intravesical and systemic chemotherapy in the management of superficial bladder cancer, Urol.Clin.North Am. 11 (4):623-635, 1984), was provided from JCRB Cell Bank and was cultured in Eagle's minimal essential medium (Life Technologies) supplemented with 10% fetal bovine serum.

### Tumor growth and treatment

1x10⁶ cells were injected subcutaneously into the flanks of mice and were allowed to grow until tumors reached a volume between 40 and 130 mm³ (around day 10-11 post tumor cell inoculation). Animals with tumors are then randomized to 4 different groups as indicated in Table 1.

**Table1: group of animals included in the study.**

| Group | Animal number | Treatment |
|---|---|---|
| 1 | 10-12 | vehicle + IgG2B (Control) |
| 2 | 10-11 | tasquinimod + IgG2B |
| 3 | 10-11 | vehicle + PD1/PDL1 antibody |
| 4 | 10-11 | tasquinimod+ PD1/PDL1 antibody |

Tumor volumes in mm³ were assessed by caliper measurements and were calculated by the formula = (Width) ²x Length /2.

As mentioned in Table 2, the mice were treated with tasquinimod (30 mg/kg) p.o. *(per os,* i.e. oral administration) twice a day. For the blockade of PDL1, 200µg of the PD1/PDL1 antibody (clone 10F.9G2; Bio-XCell) was administered i.p. (intraperitoneally) to mice every 3 days. All studies were approved by the Bioethics committee of IPSEN (CE2A) and by the French Ministry of Agriculture and Fisheries' veterinary services.

**Table 2: List of the applied treatment and the corresponding doses.**

| **Treatment** | **Reference** | **Administration per animal** |
|---|---|---|
| **PD1/PDL1 antibody** (clone 1.0F.9G2: Rat IgG2b) | BioXCell BE0101 | 200µg (i.p.) |
| **Rat IgG2b** (clone LTF-2) | BioXCell BE0090 | 200µg (i.p.) |
| **Tasquinimod (Batch 62)** | | 30mg/kg in 10ml/kg (p.o.) |
| **Vehicle** | Water (pH=8.8-9.2) | 10ml/kg in 10ml/kg (p.o.) |

### Results

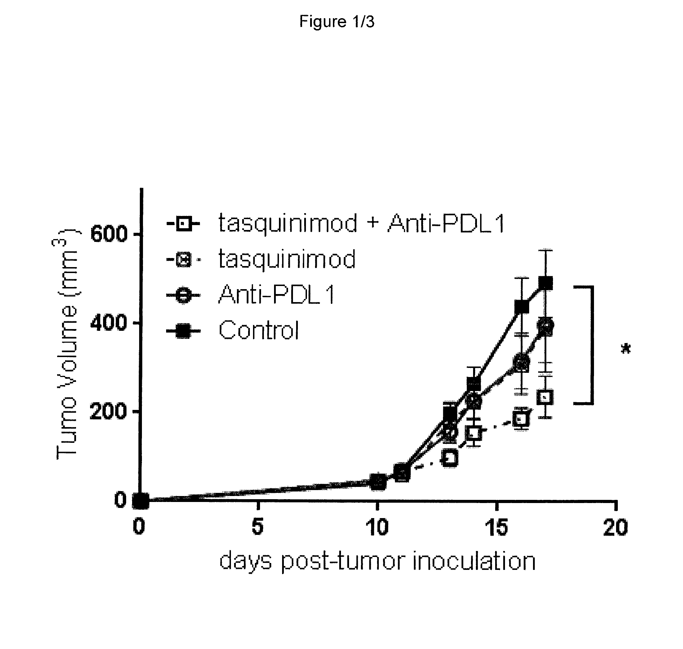

The PD1/PDL1 antibody or tasquinimod alone had a slight non-significant impact on tumor growth of MBT-2 tumors (cf. Table 3 and Figure 1).

However, treatment of mice with a combination of both compounds effectively controlled tumor growth.

**Table 3: Average of tumor volumes ± SEM (standard error of the mean) (mm³) in the different treated groups. P Values corresponds to a T.test in each group in comparison to control group.**

| | | **Days post tumor cell inoculation** | **0** | **10** | **11** | **13** | **14** | **15** | **17** |
|---|---|---|---|---|---|---|---|---|---|
| **4** | Tasquinimod + PD1/PDL1 antibody | Average SEM P | 0 | 46 | 68 | 99 | 156 | 188 | 238 |
| | | | 0 | 9 | 7 | 19 | 31 | 24 | 47 |
| | | | | 0,665 | 0,796 | 0,004 | 0,046 | 0,003 | 0,013 |
| **3** | Tasquinimod | Average SEM P | 0 | 49 | 61 | 180 | 225 | 309 | 390 |
| | | | 0 | 6 | 7 | 41 | 56 | 65 | 97 |
| | | | | 0,402 | 0,403 | 0,676 | 0,558 | 0,171 | 0,417 |
| **2** | PD1/PDL1 antibody | Average SEM P | 0 | 47 | 70 | 158 | 229 | 318 | 400 |
| | | | 0 | 7 | 9 | 25 | 38 | 63 | 85 |
| | | | | 0,550 | 0,930 | 0,247 | 0,506 | 0,194 | 0,425 |
| **1** | Control | Average SEM | 0 | 41 | 71 | 201 | 266 | 440 | 493 |
| | | | 0 | 7 | 9 | 25 | 39 | 65 | 76 |

### Conclusion

As shown above, treatment with a combination of tasquinimod and PD1/PDL1 antibody synergistically promotes antitumor activity in mice.

### Example 2: Gene expression profiling in tumors and PDL1 expression in myeloid cells derived from mice treated with tasquinimod

### Methods

### Quantitative Real-time PCR (qRT-PCR)

Mice were treated as described in Example 1. After 10 days of treatment with tasquinimod, i.e. 20 days after tumor inoculation, RNA from tumors was isolated from 100µm of OCT-embedded tumor cryosections using Trizol Reagent (LifeTechnologies). RNA concentration and purity was determined through measurement of A260/A280 ratios with a NanoDrop ND-1000 spectrophotometer. RNA integrity was checked using the Agilent 2100 Bioanalyzer. cDNA was prepared using High-Capacity cDNA Reverse Transcription Kit (LifeTechnologies) following the manufacturer's instructions. Q-PCR was performed with a three step PCR-protocol (95°C for 10 min, followed by 40 cycles of 95°C for 15 sec and 60°C for 1 min) using Taqman gene expression (LifeTechnologies). Expression levels were calculated as normalized ΔCt expression values between target gene and the two "housekeeping" and Hmbs and Cyclophilin A for Taqman technology. Data were presented as fold induction (2ΔΔCt) of treated tumors compared to control tumors. The probes used in the experiments are the following:

| **Mouse Genes** | **Assay Reference (lifetechnologies)** |
|---|---|
| II12b | Mm00434174_m1 |
| Mrc1 | Mm00485172_m1 |
| Nos2 | Mm00440502_m1 |
| Cd274 | Mm00452054_m1 |
| Ppia | Mm03302254_g1 |
| Hmbs | Mm01143545_m1 |

### FACS analysis

Mice were treated as described in Example 1. After 7 days of treatment with tasquinimod, i.e. 17 days after tumor inoculation, single cell suspensions were prepared from tumors by incubating the tumors, cut into small pieces, in 8 mg/ml Collagenase type IV (lifetechnologies) and 0.1% DNase (Sigma-Aldrich, St. Louis, MO) for 45 minutes at 37°C. Tumors were meshed in a 70 µm cell strainer. Cells were blocked with Fc-blockers (clone 2.4G2; BD Biosciences), and then stained with CD11b APC (clone M1/70) and CD274 PE Cy7 (clone MIH5) purchased from BD Biosciences (San Jose, CA) and eBioscience respectively. Cells were analyzed by Fortessa X-20 (BD Biosciences).

### Results

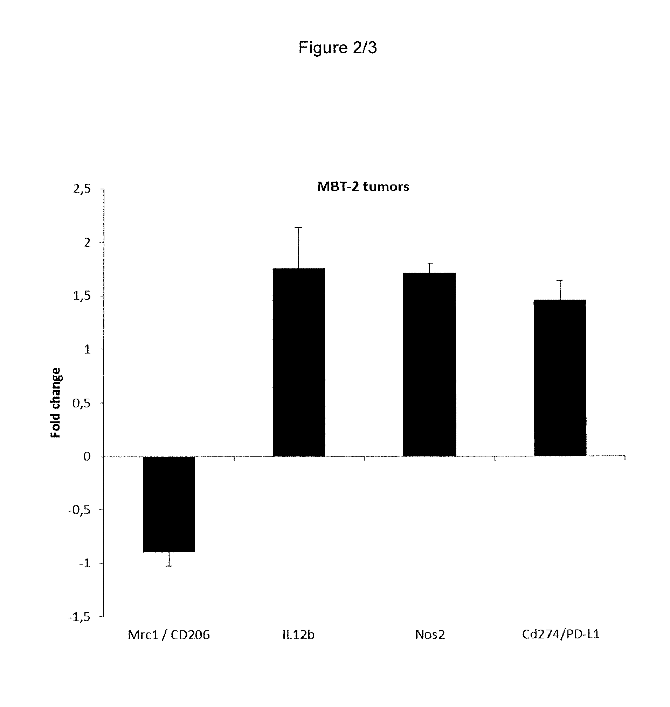

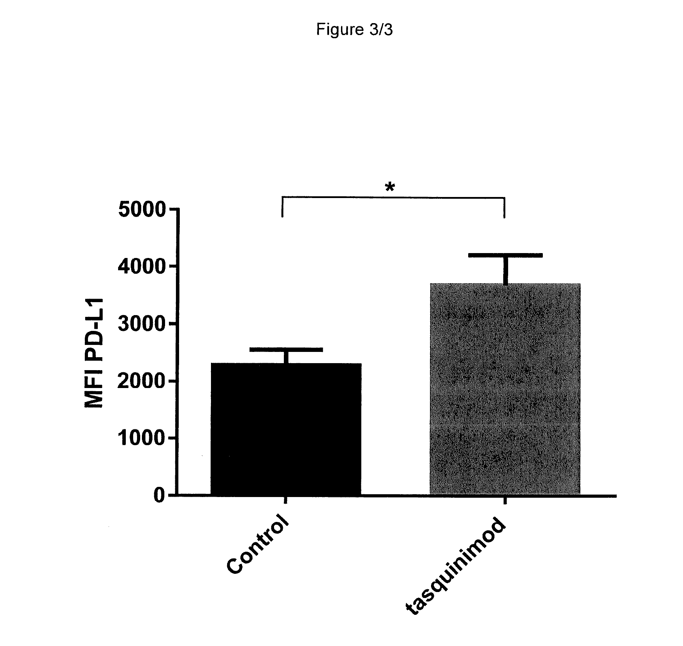

A decrease in the expression of CD206, a M2 macrophage marker, and an increase in the expression of nos2 and II-12b, both known to be released by macrophages M1, was observed (Fig. 2). This phenotype switch of macrophages from M2 to M1 induced by tasquinimod was associated with an increase in mRNA expression of Cd274 in the tumor microenvironment. Interestingly, and unexpectedly, we also found an increase in the median fluorescence intensity of PD-L1 expressed on myeloid cells of mice bearing tumors treated with tasquinimod (Fig. 3). Our findings strongly suggest that tasquinimod induces a local pro-inflammotory milieu in the tumors, which in turn may induce the expression of PD-L1. This increase of PD-L1 after tasquinimod treatment may attenuate its antitumor immune response and provides insight into the rationale for a combination of tasquinimod with Anti-PDL1 in aggressive bladder cancer to potentiate its antitumor activity.

## Claims

1. A combination comprising tasquinimod, or a pharmaceutically acceptable salt thereof, and a PD1 and/or PDL1 inhibitor, for use as a medicament.

2. A combination comprising tasquinimod, or a pharmaceutically acceptable salt thereof, and a PD1 and/or PDL1 inhibitor, for use for the treatment of cancer.

3. The combination for use according to claim 2, wherein the cancer is selected from: bladder cancer, melanoma, lung cancer, prostate cancer, renal cell carcinoma, hematologic malignancies and ovarian cancer, neuroendocrine tumors (NET) and gastroenteropancreatic neuroendocrine tumors (GEP-NET).The Combination for use according to any of claims 2 or 3, for the treatment of bladder cancer.

4. The Combination for use according to any of claims 2 to 4, for the treatment of non muscle invasive bladder cancer, muscle invasive bladder cancer or metastatic urothelial bladder cancer.

5. The combination for use according to any of the preceding claims, wherein the combination improves progression-free survival and/or life expectancy.

6. The combination for use according to any of the preceding claims, wherein tasquinimod, or a pharmaceutically acceptable salt thereof, and the PD1 and/or PDL1 inhibitor, are administered separately, sequentially or simultaneously.

7. The combination for use according to any of the preceding claims, wherein the PD1 and/or PDL1 inhibitor is a PD1 and/or PDL1 antibody.

8. Pharmaceutical composition comprising tasquinimod, or a pharmaceutically acceptable salt thereof, and a PD1 and/or PDL1 inhibitor.

9. The pharmaceutical composition according to claim 9 wherein the PD1 and/or PDL1 inhibitor is a PD1 and/or PDL1 antibody.

10. Product comprising tasquinimod, or a pharmaceutically acceptable salt thereof, and a PD1 arid/or PDL1 inhibitor, as a combined preparation for simultaneous use, separate use or use spread out over time for the treatment of cancer.

11. Combination for use according to any of claims 1 to 8, or pharmaceutical composition according to any of claims 9 to 10, or product according to claim 11, for use in the treatment of cancer, in particular bladder cancer, as a 1^{st} line treatment.

12. Combination for use according to any of claims 1 to 8, or pharmaceutical composition according to any of claims 9 to 10, or products according to claim 11, for use in the treatment of a cancer, in which cancer cells express high levels of PD1 and/or PDL1, in particular, in a bladder cancer in which cancer cells express a detectable or increased level of PD1 and/or PDL1.

13. A kit comprising tasquinimod and a package insert comprising instructions for using tasquinimod in combination with a PD1 and/or PDL1 inhibitor to treat cancer in an individual.

14. A method for treating cancer in a patient in need thereof comprising administering to the individual an effective amount of tasquinimod and a PD1 and/or PDL1 inhibitor.
